# EUROPEAN PATENT SPECIFICATION

(11) **EP 4 302 831 B1**
(45) Date of publication and mention of the grant of the patent: **27.08.2025**
(21) Application number: 22182845.2
(22) Date of filing: 04.07.2022
(51) Int. Cl.: A61P 35/00, C07D 413/12, A61K 31/5377

(54) **PROCESS FOR SYNTHESIS OF TIRBANIBULIN**
VERFAHREN ZUR SYNTHESE VON TIRBANIBULIN
PROCÉDÉ DE SYNTHÈSE DE TIRBANIBULIN

(43) Date of publication of application: 10.01.2024
(73) Proprietor: Trifarma S.p.A., 20816 Ceriano Laghetto (MB) (IT)
(72) Inventor: COLOMBO, Carlo Marino, 20162 MILANO (MI) (IT); BOTTA, Daniela, 20162 MILANO (MI) (IT); CRIPPA, Lorenzo, 20162 MILANO (MI) (IT)
(74) Representative: Bianchetti & Minoja with Trevisan & Cuonzo IPS SRL

(56) References cited:
- WO-A1-2008/082637
- WO-A2-2010/135429
- US-A1- 2021 198 202
- MICHAEL P. SMOLINSKI ET AL: "Discovery of Novel Dual Mechanism of Action Src Signaling and Tubulin Polymerization Inhibitors (KX2-391 and KX2-361)", JOURNAL OF MEDICINAL CHEMISTRY, vol. 61, no. 11, 14 June 2018 (2018-06-14), US, pages 4704 - 4719, XP055768150, ISSN: 0022-2623, DOI: 10.1021/acs.jmedchem.8b00164

## Description

The invention relates to a process for the synthesis of tirbanibulin.

### Background to the invention

Tirbanibulin, N-benzyl-2-(5-(4-(2-morpholinoethoxy)phenyl)pyridin-2-yl) acetamide of formula **I** 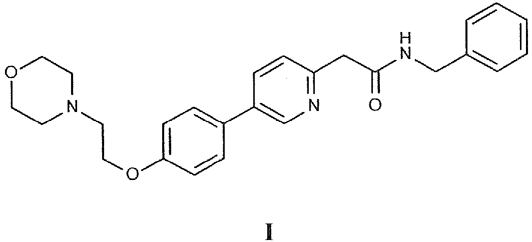 is a product used in the treatment of the actinic keratosis, a chronic recurrent skin condition caused by lengthy exposure to solar radiation.

WO 2006/071960 reports the first published synthesis for tirbanibulin. In said document the compound is identified as Compound 134 - KX2-391, and is prepared on a small scale according to the reaction reported in Scheme 1, wherein Fibrecat^{™} is polymer bound di(acetate)dicyclohexylphenylphosphine palladium(II).

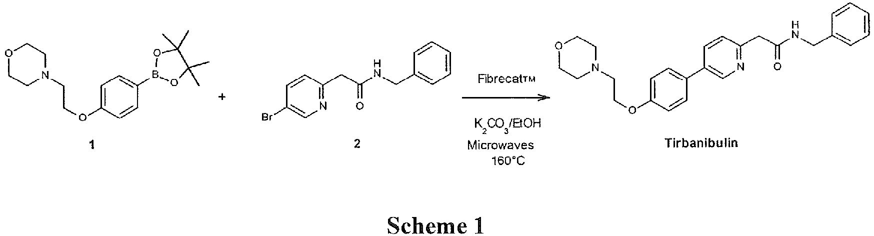

The same synthesis is reported in US 2007/0015752 and WO2008/002676.

WO2008/082637, US20080287436, WO2009051848, US20090318450 and WO2010135429 disclose the synthesis of tirbanibulin on a small scale (up to 50 g; Example 1), on an intermediate scale (at least 50 g of tirbanibulin dihydrochloride; Example 2) and on a large scale (at least 100 g of tirbanibulin dihydrochloride; Example 3). The small-scale synthesis is the same as described in Scheme 1, whereas the larger-scale synthesis follows the steps shown in Scheme 2 (which corresponds, including the numbering of the intermediates, to the scheme reported in Example 2 of WO2008/082637).

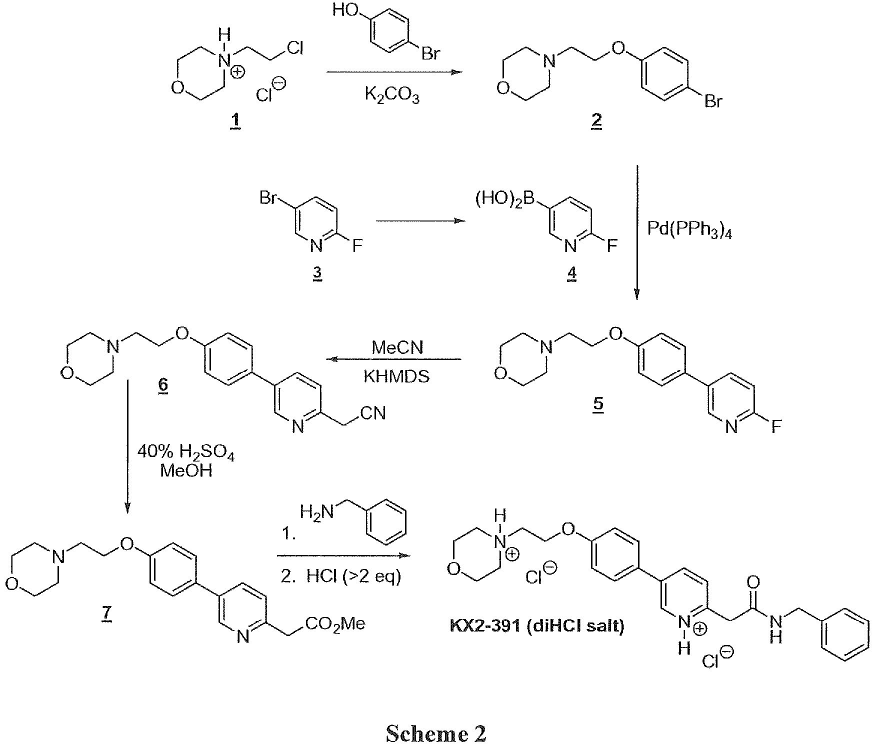

CN106810490 discloses a crystalline form of tirbanibulin. The free base is obtained by the procedure described in US7300931, which belongs to the patent family of the above-mentioned WO2006/071960.

Finally, the supporting information to the publication Journal of Medicinal Chemistry (2018), 61(11), 4704-4719, wherein tirbanibulin is identified as "compound 1 (KX2-391)", describes the synthesis reported in Scheme 3, which corresponds, including the numbering of the intermediates, to the scheme reported in the above-mentioned publication.

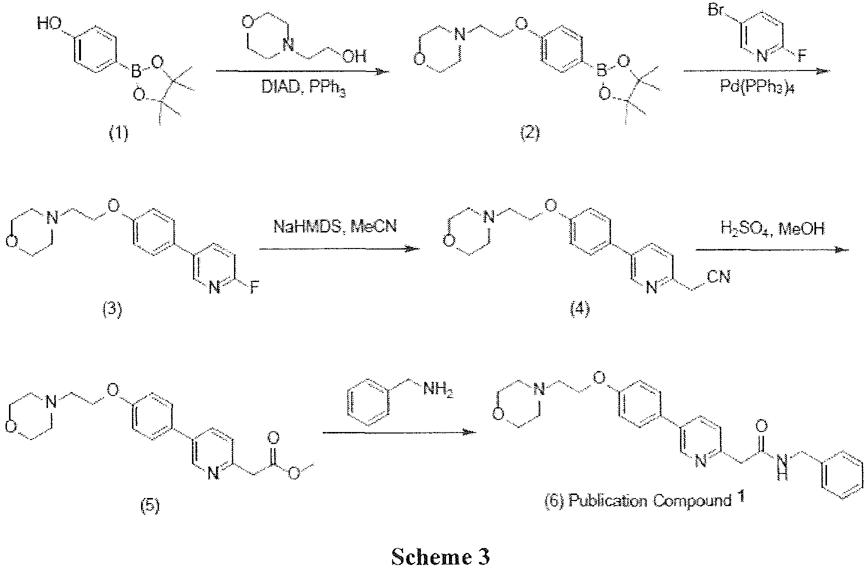

In all the known syntheses, the key reaction appears to be construction of the 3-pyridylphenyl system with a Suzuki reaction between a 3-bromopyridine derivative and a phenylboronic ester substituted at the 4 position by the 2-(4-morpholinoethoxy) group (Schemes 1 and 3), or between 5-fluoro-3-pyridylboronic acid and 4-(2-(4-morpholino)ethoxy)bromobenzene, the latter being followed by further multi-step processing to introduce the N-benzylacetamide residue.

As is clear from schemes 1-3, the known syntheses require multiple steps for the construction of the intermediates to undergo the Suzuki reaction and/or for the introduction of the N-benzylacetamide group.

A more efficient method of preparing tirbanibulin with high yields and purity therefore needs to be developed.

### Description of the invention

The object of the invention is a process for the preparation of tirbanibulin, which comprises the following steps:
a) Suzuki reaction between a compound of formula **II** 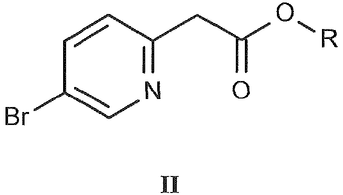
   wherein R is a (C₁-C₄)-alkyl, and 4-hydroxyphenylboronic acid of formula **III** 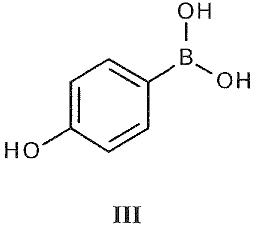 in the presence of a palladium-based catalyst and in the presence of an inorganic base or of an alkali metal fluoride, to give a compound of formula **IV** 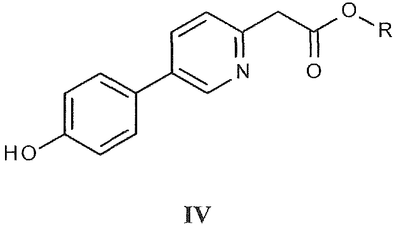
   wherein R is as defined above in a mixture of acetone/water 10/1 or dioxane/water 10/1, operating at a temperature ranging between 35°C and the boiling point of the mixture of solvents;
b) reaction of a compound of formula **IV** with a compound of formula **V** 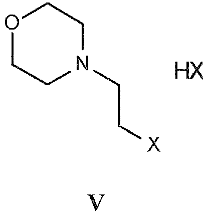 wherein X is a leaving group selected from chlorine, bromine, iodine, (C₁-C₃)-alkyl sulphonate and aryl sulphonate, preferably chlorine, to give a compound of formula **VI** 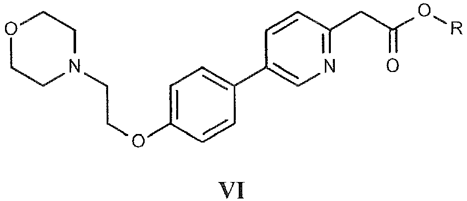 wherein R is as defined above;
c) reaction of a compound of formula **VI** with benzylamine in toluene in the presence of 2-pyridone to give tirbanibulin base of formula **I.**

The process according to the invention is illustrated in Scheme 4.

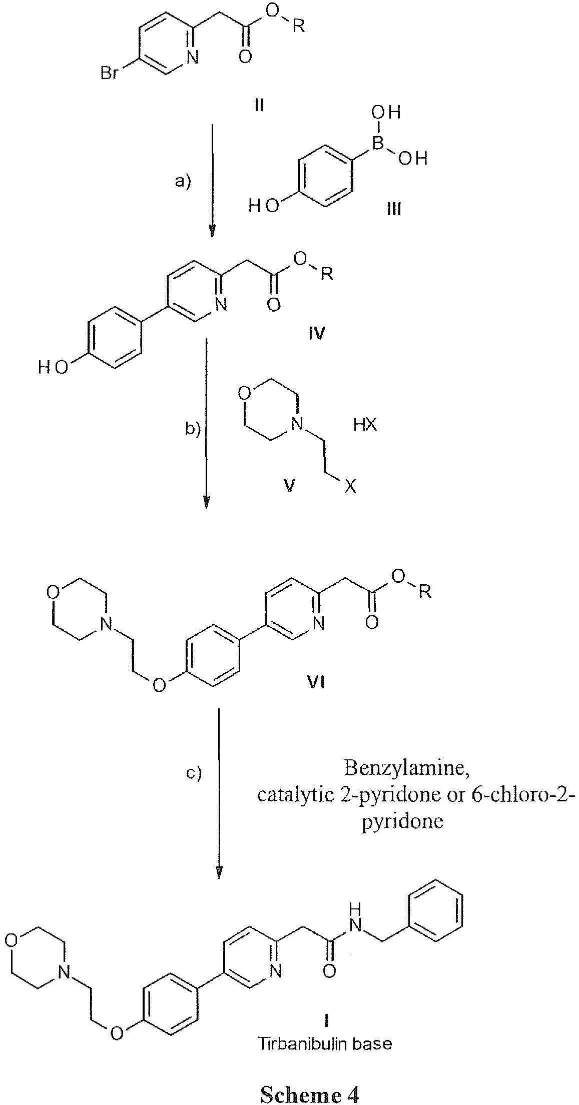

### Detailed description of the invention

A molar ratio of ester **II** to 4-hydroxyphenylboronic acid **III** ranging between 1 and 1.5, preferably 1.2, will preferably be used in step a).

The catalyst is preferably selected from Pd(OAc)₂, Pd(PPh₃)₄ and palladium on carbon (Pd/C), and is preferably used in a molar ratio ranging between 0.005 and 0.1 to compound **II.**

More preferably the catalyst is Pd(PPh₃)₄, used in a molar ratio of 0.01 to compound **II.**

The inorganic base is selected from Na₂CO₃, K₂CO₃ and Cs₂CO₃, preferably Na₂CO₃, and is used in a molar ratio ranging between 1 and 4, preferably 3, to compound **II.**

The alkali metal fluoride is preferably KF, and it is used in a molar ratio ranging between 1 and 6, preferably 3, to compound **II.**

The reaction is conducted in a mixture of acetone/water 10/1 or dioxane/water 10/1, operating at a temperature ranging between 35°C and the boiling point of the mixture of solvents.

A II/dioxane/water 1/10/1 w/v/v ratio and a **II**/acetone/water 1/10/1 w/v/v ratio are preferably used.

In specific embodiments of the invention, step a) is conducted in the presence of Pd(OAc)₂, Pd(PPh₃)₄ or Pd/C in the presence of KF in a mixture of dioxane/water under reflux.

In another specific embodiment, step a) is conducted in the presence of Pd(OAc)₂ and Na₂CO₃ in a mixture of dioxane/water under reflux.

In a preferred embodiment, step a) is conducted in the presence of Pd(PPh₃)₄ and potassium fluoride in a 10/1 mixture of dioxane/water.

The use of Pd/C as catalyst, which can be recovered at the end of the reaction, is advantageous in terms of the environmental sustainability of the process.

After conventional isolation and purifications, for example by dilution/trituration in a solvent belonging to the family of alkyl ethers, preferably in diisopropyl ether, compound **IV** is obtained with yields of around 80% and a purity suitable for use in the next step (97%).

Step b) is typically conducted at a molar ratio of compound **V** to compound **IV** ranging between 2 and 3, preferably 2.2.

The compounds of formula V are known and commercially available. In a preferred embodiment, a compound of formula **V** is N-(2-chloroethyl)morpholine hydrochloride.

The reaction is typically conducted in the presence of 2-4.5 molar equivalents to compound **IV** of an inorganic base selected from K₂CO₃ and Cs₂CO₃, preferably in the presence of 2 molar equivalents of Cs₂CO₃, and optionally in the presence of a phase-transfer catalyst.

The reaction is conducted in an alcohol-based solvent selected from methanol, ethanol and isopropanol, operating at the reflux temperature, or in dimethylformamide at a temperature not exceeding 120°C. The reaction is preferably conducted in ethanol under reflux, optionally in the presence of tetrabutyl ammonium bromide.

After conventional processing, compound **VI** is isolated with a yield of 90-100% and with suitable purity (about 90%) for use in the next step.

If the reaction is conducted in an alcohol-based solvent other than the alcohol residue present in ester **IV,** the resulting compound **VI** consists of a mixture of esters derived from partial transesterification, which can be used "as is" in the next step.

Typically, step c) is conducted using 2 to 5, preferably 3.3, molar equivalents of benzylamine to compound **VI,** in, toluene . The reaction is conducted at the reflux temperature of the solvent, in the presence of 0.1-0.5 molar equivalents to the compound of formula **VI,** preferably 0.2 molar equivalents, of 2-pyridone. The use of said catalysts allows the reaction to be conducted at lower temperatures but with reaction rates and yields comparable to those of reactions conducted in the absence thereof, using a solvent in normal industrial use such as toluene. Moreover, the crude reaction products of reactions conducted in the presence of said catalysts contain fewer by-products and/or impurities.

The crude reaction product can be purified according to the procedures described in the prior art, such as those described in WO2009/051848, to provide tirbanibulin base.

Tirbanibulin base can optionally be converted to tirbanibulin dihydrochloride according to the methods described in the prior art, for example in WO2009/051848.

Compound II is a known compound, obtainable by known methods. It is preferably obtained by esterification of 2-(5-bromopyridin-2-yl)acetic acid of formula **VII,** which is commercially available, 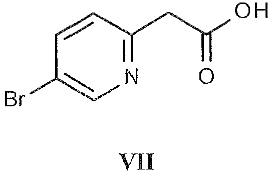 with a (C₁-C₄)- alkyl alcohol, preferably methanol, in the presence of acid catalysis, preferably with sulphuric acid.

A further object of the present invention is therefore a process for the preparation of tirbanibulin wherein a compound of formula **II** is obtained by esterification of 2-(5-bromopyridin-2-yl)acetic acid **VII,** with a C₁-C₄ alkyl alcohol, preferably methanol, in the presence of acid catalysis.

The process of the invention is characterised by steps that take place in easily controlled temperature ranges, times and reaction conditions, requiring simple purifications, and supplying the corresponding reaction products with high yields and purity.

The invention is further illustrated by the following examples.

### Examples

The ¹H and ¹³C NMR spectra were recorded with a Bruker 400 MHz instrument.

The HPLC analyses were performed with a Perkin Elmer instrument, using the method described in Example 4.

The mass spectra were recorded with a Waters Micromass ZQ quadrupole instrument.

### Example 1: Methyl 2-(5-bromopyridin-2-yl)acetate (Compound II, R = CH₃)

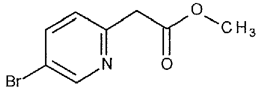

The reactions are conducted in a nitrogen atmosphere. 25 g (116 mmol) of 2-(5-bromopyridin-2-yl)acetic acid **VII** is suspended in 500 ml of methanol in a 4-necked 1-litre flask. The suspension is heated to about 30°C to obtain solubilisation, and 13.75 ml (d 1.84; 248 mmol) of 96% sulphuric acid is then cautiously added. The temperature of the reaction mixture rises spontaneously to 40°C. The mixture is heated under reflux (60-65°C) for about three hours, to obtain a complete reaction. The reaction volume is reduced to about a third of the initial volume, and 250 ml of dichloromethane and about 750 ml of a 5% aqueous solution of potassium bicarbonate are added until a pH of between 8 and 8.5 is reached. The phases are separated and the aqueous phase re-extracted with 2 x 200 ml of dichloromethane. The combined organic phases are washed with 1 x 200 ml of a 5% aqueous solution of potassium bicarbonate, and then with 1 x 200 ml of water. The mixture is dried over sodium sulphate and filtered, and the solvent distilled, to obtain 24.7 g of methyl ester as oil (107 mmol; yield 93%).

Following the same procedure, but using ethanol instead of methanol, ethyl 5-bromopyridin-2-acetate (compound **II,** R = CH₂CH₃) is prepared.

### Example 2: Methyl 2-[5-(4-hydroxyphenyl)-pyridin-2-yl]acetate compound IV, R = CH₃)

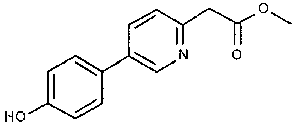

The reaction is conducted in a nitrogen atmosphere, using solvents thoroughly degassed by bubbling nitrogen through them. 24.0 g (104.32 mmol) of methyl 2-(5-bromopyridin-2-yl)acetate, 240 ml of 1,4-dioxane (degassed) and 24 ml of purified water (degassed) are loaded into a 4-necked 500 ml flask. 15.85 g (114.9 mmol) of 4-hydroxyphenylboronic acid **III**, 18.1 g (311.53 mmol) of KF and 1.2 g (1.04 mmol) of Pd(PPh₃)₄ are added to the resulting solution. The mixture is left under stirring under reflux for about 4 hours; the reaction is biphasic. A slight excess of 4-hydroxyphenylboronic acid (1 g, 7.25 mmol) is added. A second excess of 4-hydroxyphenylboronic acid (0.5 g, 3.62 mmol) is added after 1 hour, and the reaction is left to continue for a further 3 hours. The mixture is cooled to room temperature, the phases are separated, and the aqueous phase is re-extracted with 2 x 100 ml of dichloromethane + 1% dioxane. 250 ml of dichloromethane and 250 ml of water are added to the combined organic phases. The phases are separated, and the organic phase dried over sodium sulphate. The organic phase is filtered and distilled at low pressure to obtain a dark yellow solid, which is broken up in 200 ml of diisopropyl ether. The solid is filtered, washed with 2 x 20 ml of diisopropyl ether, and dried under vacuum at 45°±5°C.
19.7 g (yield: 78%) of product IV (R = CH₃) is obtained, with 97% HPLC purity if the method described in Example 4 is used.
400 MHz ¹H NMR, DMSO-d₆ (δ, ppm): 9,66 (1H, s), 8,72 (1H, d), 7,94 (1H, dd), 7,54 (2H, d), 7,38 (1H, d), 6,89 (2H, d), 3,87 (2H, s), 3,63 (3H, s).
100 MHz, ¹³C NMR, DMSO-d₆ (δ, ppm): 171,7 (CO), 158,5 (C), 153,2 (C), 147,25 (CH), 134,8 (C), 134,6 (CH), 128,7 (CH), 128,3 (C), 124,7 (CH), 116,75 (CH), 52,5 (CH₃), 43,4 (CH₂).
ESI-MS: m/z 244 [M+H]⁺

### Example 3: Methyl-2-(5-(4-(2-morpholinoethoxy)phenyl)pyridin-2-yl)acetate (compound VI, R = CH₃)

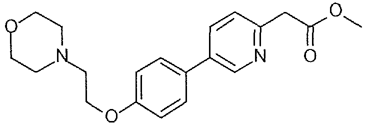

The reaction is conducted in a nitrogen atmosphere. 19.5 g (80.2 mmol) of methyl 2-[5-(4-hydroxyphenyl)-2-pyridyl]acetate in 400 ml of ethanol is loaded into a 4-necked 1-litre flask. 32.4 g (174.2 mmol) of 4-(2-chloroethyl)morpholine hydrochloride, 51.6 g (158.4 mmol) of caesium carbonate and 0.2 g (0.62 mmol) of tetrabutylammonium bromide are added. The mixture is heated to reflux. After 3 hours under reflux, the reaction is completed and the formation of two products due to partial transesterification is observed in HPLC (method indicated in Example 4). The caesium carbonate is filtered. The solvent is distilled at low pressure, and the residue (dark orange oil) is taken up with 250 ml of ethyl acetate. The organic phase is washed with 3 x 200 ml of water. After phase separation, the organic phase is decoloured with 1 g of carbon, heating it in an inert atmosphere at 50-60°C for 30-60 minutes. The carbon is filtered off, the filtrate is dried over sodium sulphate, the sodium sulphate is filtered, and the solvent is distilled at low pressure. The residue is taken up with heptane (120 ml) and the solvent is distilled at low pressure, to obtain a yellow oil deemed sufficiently pure to be used "as is" in the next step.

The conversion is deemed to be 100%, as the weight of the oil exceeds the theoretical value (28.6 g) due to the presence of residual solvent.

HPLC analysis conducted by the method described in Example 4 shows 90% purity, considered sufficient for the next step.
400 MHz ¹H NMR, CDCl₃ (δ, ppm): 8,78 (1H, d), 7,85 (1H, dd), 7,53 (2H, d), 7,37 (1H, d), 7,04 (2H, d), 4,20 (2H, t), 3,92 (2H, s), 3,78 (7H, m), 2,87 (2H, t), 2,63 (4H, m).
100 MHz ¹³C NMR, CDCl₃) (δ, ppm): 170,8 (CO), 158,5 (C), 152,1 (C), 147,2 (CH), 134,3 (C), 134,2 (CH), 129,8 (C), 127,8 (CH), 123,3 (CH), 114,8 (CH), 66,5 (CH₂), 65,6 (CH₂), 57,2 (CH₂), 53,7 (CH₂), 51,8 (CH₃), 43,0 (CH₂).
ESI-MS: m/z 357 [M+H]⁺

### Example 4: N-benzyl-2-(5-(4-(2-morpholinoethoxy)phenyl)pyridin-2-yl) acetamide (tirbanibulin, I)

The reaction is conducted in a nitrogen atmosphere. The product obtained according to Example 3, deemed to contain 28.6 g (80 mmol) of methyl-2-(5-(4-(2-morpholinoethoxy)phenyl)pyridin-2-yl)acetate, dissolved in 230 ml of toluene, is loaded into a 4-necked 500 ml flask. 28.6 g (267 mmol) of benzylamine and 1.50 g (16 mmol) of 2-pyridone are added. The mixture is heated under reflux for 26-28 hours. The solvent is distilled at low pressure until a solid residue is obtained, which is taken up by dissolving it with 250 ml of dichloromethane. The organic phase is repeatedly washed with water (6 x 175 ml). The phases are separated and the organic phase is decoloured by adding 1.5 g of carbon and leaving it under stirring for 30-60 minutes at room temperature. The carbon is filtered, and the filtrate is dried over sodium sulphate and filtered. The dichloromethane is distilled to obtain a solid residue, which is taken up with 71.5 ml of toluene. The mixture is left under stirring to break it up at room temperature for 30-45 minutes, and 45 ml of a toluene:heptane 1:1 solution is then added. The mixture is left under stirring at room temperature for about 30-45 minutes; 30 ml of heptane is then added, and the resulting mixture is left under stirring at room temperature for about 30-45 minutes. The broken-up solid is filtered and washed, first with toluene:heptane 2:1 (30 ml), next with toluene:heptane 1:2 (30 ml), and finally with heptane (20 ml), as described in WO2010/135429. After stove-drying under vacuum at 45-50°C, 22 g of tirbanibulin **I** is obtained in the form of a straw-coloured solid.
Yield: 64% purity 95%
400 MHz ¹H NMR, CDCl₃ (δ, ppm): 8,74 (1H, d), 7,87 (1H, dd), 7,80(1H, br t), 7,54 (2H, d), 7,45-7,20 (6H, m), 7,07 (2H, d), 4,54 (2H, d), 4,23 (2H, t), 3,87 (2H, s), 3,81 (4H, m), 2,90 (2H, t), 2,66 (4H, m).
100 MHz, ¹³C NMR, CDCl₃ (δ, ppm): 169,65 (CO), 159,3 (C), 154,0 (C), 147,4 (CH), 138,7 (C), 135,35 (CH), 135,0 (C), 130.2 (C), 128,9 (CH), 128,45 (CH), 127,9 (CH), 127,6 (CH), 124,3 (CH), 115,6 (CH), 67,2 (CH₂), 66,3 (CH₂), 57,9 (CH₂), 54,4 (CH₂), 45,2 (CH₂), 43,8 (CH₂).
ESI-MS: m/z 432 [M+H]⁺

The HPLC analysis is performed by the following method:

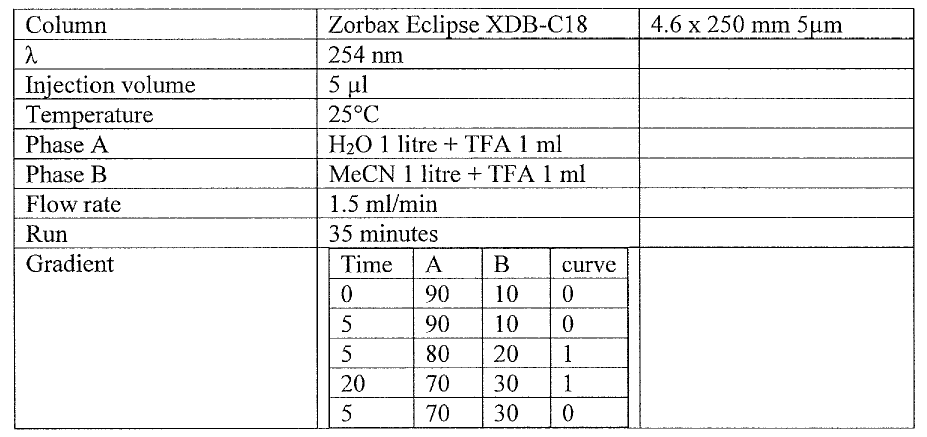

## Claims

1. A process for the preparation of tirbanibulin of formula **I** 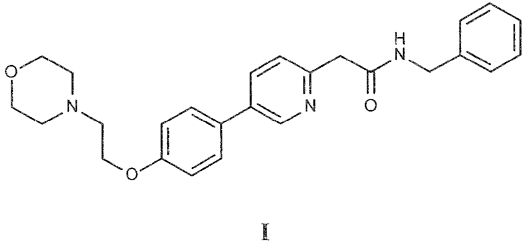 comprising the following steps:
a) Suzuki reaction between a compound of formula **II** 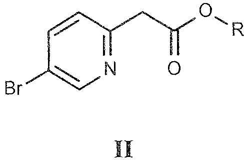
wherein R is a (C₁-C₄)-alkyl, and 4-hydroxyphenylboronic acid of formula **III** 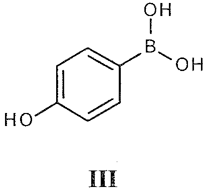
in the presence of a palladium-based catalyst and in the presence of an inorganic base or of an alkali metal fluoride, to give a compound of formula **IV** 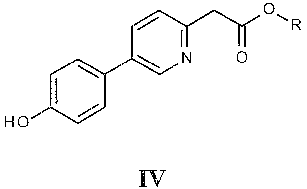
wherein R is as defined above in a mixture of acetone/water 10/1 or dioxane/water 10/1, operating at a temperature ranging between 35°C and the boiling point of the mixture of solvents;
b) reaction of a compound of formula **IV** with a compound of formula **V** 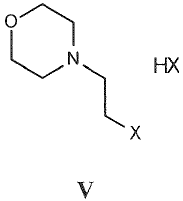
wherein X is selected from chlorine, bromine, iodine, (C₁-C₃)-alkyl sulphonate and aryl sulphonate, preferably chlorine, to give a compound of formula **VI** 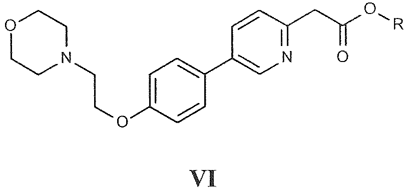
wherein R is as defined above;
c) reaction of a compound of formula **VI** with benzylamine in toluene in the presence of 2-pyridone a to give tirbanibulin of formula **I.**

2. The process as claimed in claim 1, wherein in step a), the palladium-based catalyst is selected from Pd(OAc)₂, Pd(PPh₃)₄ and palladium on carbon (Pd/C), the inorganic base is selected from Na₂CO₃, K₂CO₃ and Cs₂CO₃, and the alkali metal fluoride is KF.

3. The process as claimed in claim 2 wherein the catalyst is Pd(PPh₃)₄

4. The process as claimed in claim 3 wherein step a) is conducted in the presence of Na₂CO₃.

5. The process as claimed in claim 3 wherein step a) is conducted in the presence of KF.

6. Process as claimed in any one of the preceding claims wherein a compound of formula **V** is N-(2-chloroethyl)morpholine hydrochloride.

7. Process as claimed in any one of the preceding claims wherein a compound of formula **II** is obtained by esterification of 2-(5-bromopyridin-2-yl)acetic acid of formula **VII** 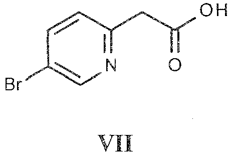 with a (C₁-C₄)-alkyl alcohol in the presence of acid catalysis.

8. The process as claimed in claim 7 wherein the (C₁-C₄)-alkyl alcohol is methanol.

## Patentansprüche

1. Verfahren zur Herstellung von Tirbanibulin der Formel **I** 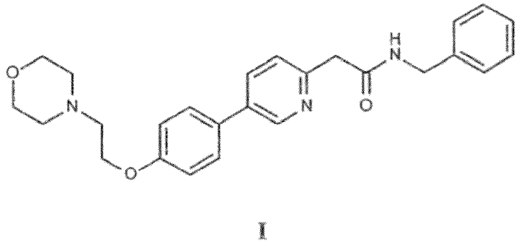 umfassend die folgenden Schritte:
a) Suzuki-Reaktion zwischen einer Verbindung der Formel **II** 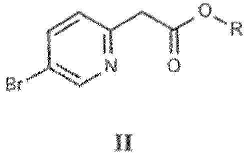
wobei R ein (C₁-C₄)-Alkyl ist, und 4-Hydroxyphenylboronsäure der Formel **III** 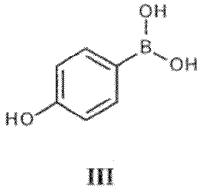
in Gegenwart eines Katalysators auf Palladiumbasis und in Gegenwart einer anorganischen Base oder eines Alkalimetallfluorids, um eine Verbindung der Formel **IV** zu ergeben 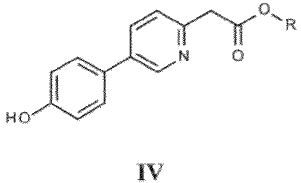
wobei R wie oben definiert ist, in einer Mischung aus Aceton/Wasser 10/1 oder Dioxan/Wasser 10/1, bei einer Temperatur zwischen 35°C und dem Siedepunkt des Lösungsmittelgemischs;
b) Umsetzen einer Verbindung der Formel **IV** mit einer Verbindung der Formel **V** 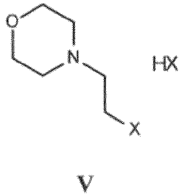
wobei X ausgewählt ist aus Chlor, Brom, lod, (C₁-C₃)-Alkylsulfonat und Arylsulfonat, vorzugsweise Chlor, um eine Verbindung der Formel **VI** zu ergeben 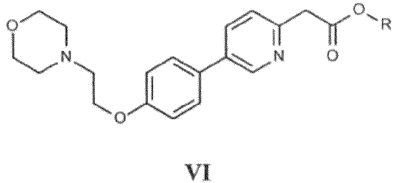
wobei R wie oben definiert ist;
c) Umsetzen einer Verbindung der Formel **VI** mit Benzylamin in Toluol in Gegenwart von 2-Pyridon a, um Tirbanibulin der Formel I zu ergeben.

2. Verfahren nach Anspruch 1, wobei in Schritt a) der Katalysator auf Palladiumbasis ausgewählt ist aus Pd(OAc)₂, Pd(PPh₃)₄ und Palladium auf Kohlenstoff (Pd/C), die anorganische Base ausgewählt ist aus Na₂CO₃, K₂CO₃ und Cs₂CO₃ und das Alkalimetallfluorid KF ist.

3. Verfahren nach Anspruch 2, wobei der Katalysator Pd(PPh₃)₄ ist.

4. Verfahren nach Anspruch 3, wobei Schritt a) in Gegenwart von Na₂CO₃ durchgeführt wird.

5. Verfahren nach Anspruch 3, wobei Schritt a) in Gegenwart von KF durchgeführt wird.

6. Verfahren nach einem der vorhergehenden Ansprüche, wobei eine Verbindung der Formel **V** N-(2-Chlorethyl)morpholinhydrochlorid ist.

7. Verfahren nach einem der vorhergehenden Ansprüche, wobei eine Verbindung der Formel **II** durch Veresterung von 2-(5-Brompyridin-2-yl)essigsäure der Formel **VII** 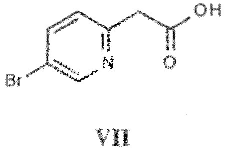 mit einem (C₁-C₄)-Alkylalkohol in Gegenwart von Säurekatalyse erhalten wird.

8. Verfahren nach Anspruch 7, wobei der (C₁-C₄)-Alkylalkohol Methanol ist.

## Revendications

1. Procédé destiné à la préparation de tirbanibuline répondant à la formule I 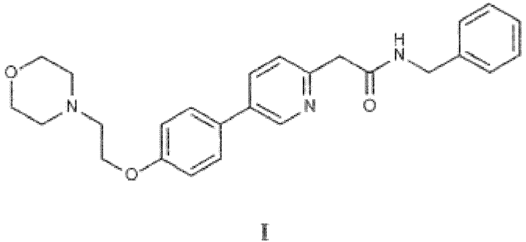 qui comprend les étapes suivantes :
a) une réaction de Suzuki entre un composé répondant à la formule **Il** 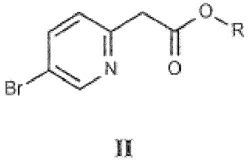
dans laquelle R représente un groupe alkyle en C₁-C₄, et un acide 4-hydroxyphénylborique répondant à la formule **III** 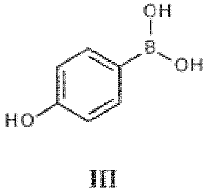
en présence d'un catalyseur à base de palladium et en présence d'une base inorganique ou d'un fluorure de métal alcalin, afin d'obtenir un composé répondant à la formule **IV** 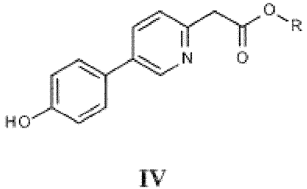
dans laquelle R est tel que défini ci-dessus dans un mélange acétone/eau 10/1 ou dioxane/eau 10/1, en travaillant à une température se situant dans la plage entre 35 °C et le point d'ébullition du mélange de solvants ;
b) la réaction d'un composé répondant à la formule **IV** avec un composé répondant à la formule **V** 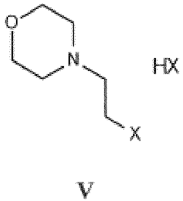
dans laquelle X est choisi parmi un atome de chlore, un atome de brome, un atome d'iode, un sulfonate d'alkyle en C₁-C₃ et un sulfonate d'aryle, de préférence un atome de chlore, afin d'obtenir un composé répondant à la formule **VI** 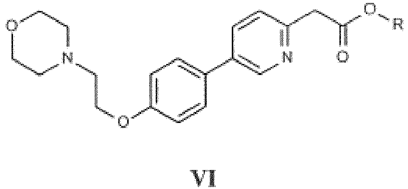
dans laquelle R est tel que défini ci-dessus ;
c) la réaction d'un composé répondant à la formule **VI** avec de la benzylamine dans du toluène en présence de la 2-pyridone afin d'obtenir la tirbanibuline répondant à la formule **I**.

2. Procédé selon la revendication 1, dans lequel, à l'étape a), le catalyseur à base de palladium est choisi parmi le Pd(OAc)₂, le Pd(PPh₃)₄ et du palladium sur du charbon (Pd/C) , la base inorganique est choisie parmi du Na₂CO₃, du K₂CO₃ et du CS₂CO₃, et le fluorure de métal alcalin est du KF.

3. Procédé selon la revendication 2, dans lequel le catalyseur est du Pd(PPh₃)₄.

4. Procédé selon la revendication 3, dans lequel l'étape a) est mise en œuvre en présence de Na₂CO₃.

5. Procédé selon la revendication 3, dans lequel l'étape a) est mise en œuvre en présence de KF.

6. Procédé selon l'une quelconque des revendications précédentes, dans lequel un composé répondant à la formule **V** est le chlorhydrate de N-(2-chloro-éthyl)morpholine.

7. Procédé selon l'une quelconque des revendications précédentes, dans lequel on obtient un composé répondant à la formule **II** par estérification de l'acide 2-(5-bromopyridin-2-yl)acétique répondant à la formule **VII** 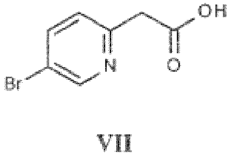 avec un alcool d'alkyle en C₁-C₄ en présence d'une catalyse acide.

8. Procédé selon la revendication 7, dans lequel l'alcool d'alkyle en C₁-C₄ est le méthanol.
